# EUROPEAN PATENT APPLICATION

(11) **EP 1 249 445 A1**
(43) Date of publication of application: **16.10.2002**
(21) Application number: 01201368.6
(22) Date of filing: 13.04.2001
(51) Int. Cl.: C07C 67/347, C07C 69/716, C07C 45/50

(54) **Continuous process for producing an aldehyde**

(71) Applicant: DSM N.V., 6411 TE Heerlen (NL)
(72) Inventor: Sielcken, Otto Erik, 6136 BG Sittard (NL); Smits, Hubertus Adrianus, 6212 GC Maastricht (NL); Toth, Imre, 6166 GM Geleen (NL)

(57) **Abstract**

A continuous process for producing an aldehyde comprising exposing an olefinically unsaturated compound to a mixture comprising carbon monoxide, hydrogen and a rhodium-organobisphosphite complex catalyst, wherein said mixture contains less than:
a) 1000 part per million vinyl cyclohexene,
b) 25 parts per million butadiene,
c) 25 parts per million of other multi-unsaturated conjugated organic compounds,
d) 100 parts per million water,
e) 100 parts per million peroxide,
f) 2 percent by weight methyl-2-methyl-2-butenoate,
g) 100 parts per million primary alcohol,
h) 100 parts per million oxygen, and
i) 10 parts per million halogen.

## Description

The invention relates to a continuous process for producing an aldehyde comprising exposing an olefinically unsaturated compound to a mixture comprising carbon monoxide, hydrogen and a rhodium-organobisphosphite complex catalyst.

It is well known in the art that aldehydes may be readily produced by reacting an olefinically unsaturated compound with carbon monoxide and hydrogen in the presence of a rhodium-organophosphorous ligand complex catalyst and that preferred processes involved continuous hydroformylation and recycling of the catalyst solution such as disclosed, for example, in US Patent Nos. 4,148,830; 4,717,775; and 4,769,498. Organophosphites, especially organobisphosphites, have proven to be among the ligands of choice in rhodium catalyzed hydroformylation reactions because such complexes exhibit exceptional activity and regioselectivity in this reaction. For instance, U.S. Pat. Nos. 4,668,651 and 4,769,498 fully detail such hydroformylation.

However, although rhodium catalyzed hydroformylation reactions have enjoyed a wealth of commercial development, efficiency and cost associated with these processes remain a primary concern, especially in light of the scarcity and high price of rhodium metal and the cost of replacing degraded organophosphorous compounds. Traditional attempts to improve the reaction rate, as well as reduce costly decomposition pathways of the metal complex catalysts have focused on ligand choice, as described above, or reaction conditions. For example, US Patent Nos. 5,288,918 and 5,763,670 describe catalyst protection and increased longevity through the addition of additives, or manipulation of the reaction conditions.

However, the rate of the hydroformylation reaction is often reduced by the presence of contaminants in the reaction mixture. Many of these contaminants react with the rhodium catalyst or organobisphosphite ligand to cause the partial decomposition of the desired catalytically active species. As a result of the irreversible chemical reaction of these contaminants with the rhodium catalyst or organobisphosphite ligand, the precious rhodium metal and ligand are destroyed and the utility of the rhodium-organobisphosphite complex as a hydroformylation catalyst is prevented. Included in this class of contaminants are oxygen, water, alcohols, strong Brönsted acids and organic peroxides.

Still other contaminants may react with the rhodium catalyst or catalyst precursors to form less reactive rhodium species. These compounds act as a type of poison towards the metal center by complexing to it with a strength that is competitive with the desired substrates such as olefinically unsaturated compounds, hydrogen or carbon monoxide. Such competitive reactions produce rhodium species which do not catalyze the desired hydroformylation reaction and therefore cause the rate of the hydroformylation reaction to decrease. This decreased rate can only be compensated for by the use of greater amounts of rhodium and, in turn, increased cost of the hydroformylation process. Included in this class of contaminants are: compounds containing at least two alkene functionalities, such as alkadienes, alkatrienes. Especially the conjugated alkadienes and alkatrienes are harmful under normal hydroformylation conditions. Examples thereof are 1,3-butadiene, 1-vinyl cyclohexene, 1,3,7-octatriene.

Also undesired is the presence of components which have a moiety as shown below, 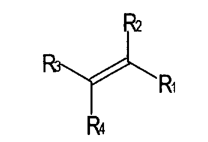 i.e. a moiety where branching in the carbon chain occurs at the alkene functionality, i.e. not more than one of the groups R₁ through R₄ is a hydrogen atom and the other groups connect to the C=C bond by a C-C bond. An example of such a component is methyl-2-methyl-2-butenoate. Although methyl-2-methyl-2-butenoate is not a catalyst poison, it is not a desired component because it does not react under normal hydroformylation conditions, it is difficult to separate and accumulates in the hydroformylation reaction mixture. On the contrary, methyl-2-methyl-3-butenoate is not a problem as this last compounds hydroformylates readily.

Since many of the alkenes and other reactants which are used commercially in the hydroformylation process contain these impurities, these catalyst poisons are often present in typical hydroformylation processes. As a result, much rhodium metal is prevented from participating in the hydroformylation process, which corresponds to increased production costs for aldehydes produced by these processes. Therefore, a method by which these catalyst poisons could be prevented from interfering with the rhodium catalyst would be a great improvement in the hydroformylation process.

The object of the invention is an improved rhodium-organophosphite complex catalyzed hydroformylation process.

This object is achieved in that said mixture contains less than:
a) 1000 part per million vinyl cyclohexene,
b) 25 parts per million butadiene,
c) 25 parts per million of other multi-unsaturated conjugated organic compounds,
d) 100 parts per million water,
e) 100 parts per million peroxide,
f) 2 percent by weight methyl-2-methyl-2-butenoate,
g) 100 parts per million primary alcohol,
h) 100 parts per million oxygen, and
i) 10 parts per million halogen.

It has now been discovered that catalyst deactivation resulting from the presence of contaminants in the reactants or solvent of a rhodium-organophosphite complex catalyzed continuous hydroformylation processes may be prevented or minimized by first purifying the reactants and solvent used in the process.

In a preferred embodiment of the present invention the rhodium-bisphosphite complex catalyst comprises a bisphosphite ligand of a formula selected from the group consisting of: 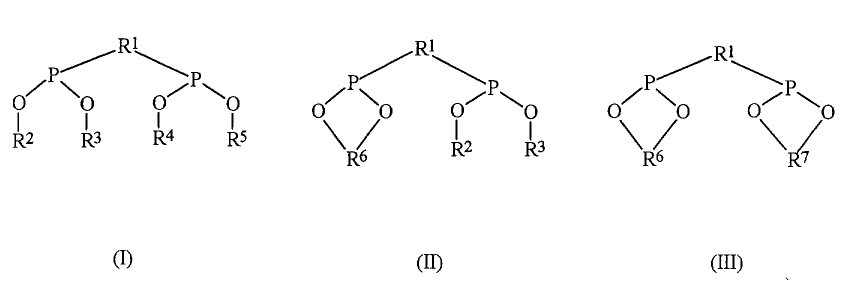 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different; wherein each Q individually represents a divalent bridging group of ―O― or ―CR'R"― wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 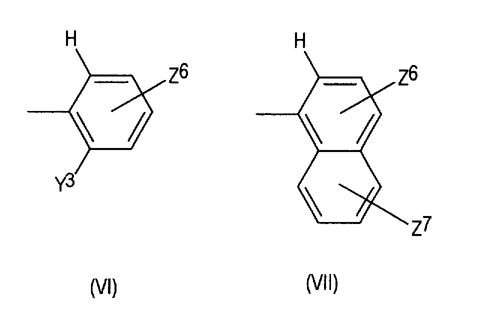 wherein R⁶ and R⁷ might be the same or different and each is individually represented by the structure of (VIII) or (IX), 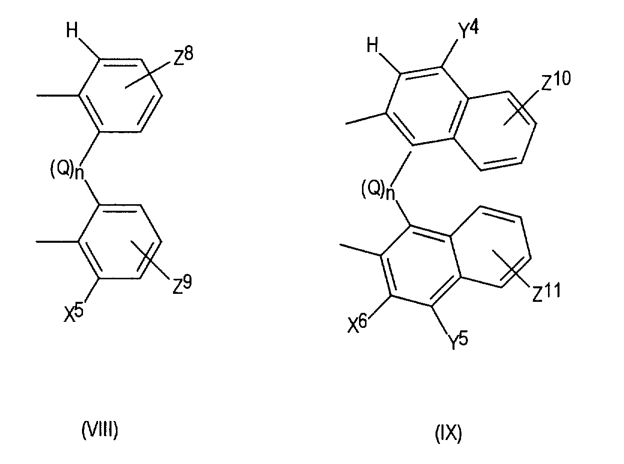 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical, wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

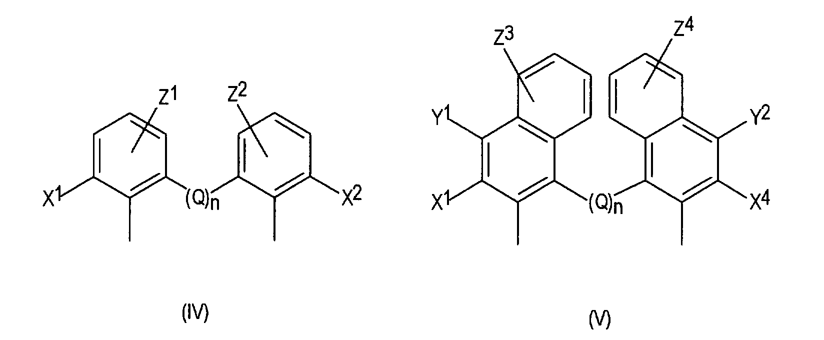

In a more preferred embodiment of the present invention R¹ is represented by the structure of (IV), (V), (VII), (VIII0, (IX), wherein (Q)ₙ is the same as above, wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).
Still more preferably said hydroformylation process is performed wherein said ligand used is chosen from the group consisting of [3,3'-bis(t-butyl)-5,5'-dimethoxy-1,1'-biphenyl-2,2'-diyl]-bis(oxy)]-bis(dibenzo[d,f] [1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

In a preferred embodiment of the invention, the mixture comprises less than
a) 500 part per million vinyl cyclohexene,
b) 10 parts per million butadiene,
c) 10 parts per million of other multi-unsaturated conjugated organic compounds,
d) 50 parts per million water,
e) 50 parts per million peroxide,
f) 1 per cent by weight methyl-2-methyl-2-butenoate,
g) 50 parts per million primary alcohol, and
h) 50 parts per million oxygen.

In a more preferred embodiment of the invention, the mixture comprises less than
a) 100 part per million vinyl cyclohexene,
b) 1 parts per million butadiene,
c) 1 parts per million of other multi-unsaturated conjugated organic compounds,
d) 10 parts per million water,
e) 10 parts per million peroxide,
f) 0.5 per cent by weight methyl-2-methyl-2-butenoate,
g) 10 parts per million primary alcohol, and
h) 10 parts per million oxygen.

In a preferred embodiment of the present invention, the olefinically unsaturated compound is percolated prior to its introduction into the hydroformylation process through one or more compounds selected from the following class: alumina, silica, and molecular sieves. In still another embodiment the water or a primary alcohol is removed from the olefinically unsaturated compound by distillation.

In another preferred embodiment of the present invention, a solvent is percolated prior to its introduction into the hydroformylation process through one or more compounds selected from the following class: aluminum oxide, silica, and molecular sieves.

In yet another embodiment of the present invention, the carbon monoxide and/or hydrogen reactants are passed through one or more members of the following class of compounds prior to its introduction into the hydroformylation process: Aluminum oxide, silica, molecular sieves and copper type catalyst beds.

In another embodiment of the present invention, the olefinically unsaturated compound is first distilled from a mixture of olefinically unsaturated compound and optionally a purifying agent selected from the class consisting of: aluminum oxide, silica, phosphorous pentaoxide, or a phosphorous compound.

Removing or at least reducing the amount of peroxide is preferably carried out as described in for example EP-A-662468.

The present invention provides an enhanced continuous hydroformylation process for producing aldehydes which comprises reacting an olefinically unsaturated compound with carbon monoxide and hydrogen in the presence of a rhodium-organobisphosphite catalyst; wherein the process comprises first purifying the reactants and solvent.

Accordingly, the subject invention encompasses preventing or minimizing the catalyst deactivation associated with poisoning of rhodium-phosphite complex catalyzed hydroformylation processes for producing aldehydes, by first purifying the reactants and solvent used in the process. By purification is meant the process by which contaminants are removed from reactants and/or solvent. Specifically the purification procedures of this invention are targeted towards the removal, from both the reactants and any solvent which may be present, of alcohols, oxygen, peroxides, unsaturated alkene compounds with tertiary carbon atom adjacent to the alkene functionality, water, and unsaturated organic compounds containing at least two alkene functionalities such as alkadienes, alkatrienes and the like.

As stated above, the subject invention resides in the discovery that removal of the above identified contaminants prevents or minimizes the deactivation of the rhodium-organophosphite catalyst. Since these contaminants can be introduced from a variety of sources, several purification procedures may be required for their removal.

Among the most harmful contaminants to the rhodium-organobisphosphite catalyzed hydroformylation process are oxygen, primary alcohols and water. The introduction of these compounds is thought to occur when the olefinically unsaturated compound, synthesis gas, or solvent is introduced into the hydroformylation process. Accordingly purification of one or more of these substances is necessary to avoid catalyst deactivation. The removal of water or a primary alcohol must be conducted in a manner so that the reaction mixture does not contain more than 100 parts per million water or primary alcohol. More preferably such a removal would limit the presence of water or a primary alcohol within the reaction mixture to 50 parts per million for each compound, and still more preferably to no more than 10 parts per million, independently, of each compound. By reaction mixture it is meant the liquid medium which is present in the hydroformylation process. The term parts per million is understood to mean the ratio consisting of grams of the specified substance per one million grams of the reaction mixture.

One of the most problematic sources of water and primary alcohols within the hydroformylation process is the olefinically unsaturated compound itself. Removal of contaminating water or primary alcohol from this source may be accomplished by a variety of means such as distillation, distillation in the presence of a purification agent, or percolation of the olefinically unsaturated compound through a column of a substance capable of absorbing water or a primary alcohol.

The term distillation, as used herein, is understood to mean any process by which two or more compounds may be separated based on the differences in their boiling point. Distillation through a purification agent is a process by which the compound to be distilled is exposed to a compound capable of reacting with or absorbing water or a primary alcohol. Such compounds may include molecular sieves, aluminum oxide, silica, phosphorous pentaoxide, or the like. The final purification technique available for removal of water from the olefinically unsaturated compound consists of percolating said olefinically unsaturated compound, in either the gas or liquid phases, through a agent capable of absorbing water. Such agents include alumina, silica, or molecular sieves.

Water and primary alcohols may also contaminate the solvent. These contaminants may be removed from the solvent in the same manner described above such as by distillation, distillation in the presence of a purification agent, or by percolation through a substance which absorbs primary alcohols and water.

Additionally, water and primary alcohols may also contaminate the syn-gas and especially the syn-gas which is recycled in this hydroformylation process. The term syn-gas, as used in this invention is understood to mean a gaseous mixture comprising hydrogen and carbon monoxide. Purification of the syn-gas to remove these contaminants may be accomplished by drying the syn-gas, by passing the syn-gas through a column of a substance which absorbs water or primary alcohols, or by passing the syn-gas over a catalyst which removes water by chemical reaction. Substances suitable for the absorption of water or primary alcohols are the same as those described above.

Of course, it is understood that any combination of above described techniques may be used to purify the olefinically unsaturated compound, syn-gas, or solvent. It is also further understood that any combination of olefinically unsaturated compound, syn-gas or solvent may be purified so long as the concentration of water or primary alcohol within the reaction mixture is no more than 100 parts per million. More preferably the concentration of water and primary alcohol is, independently, no greater than 50 parts per million, and still more preferably to no more than 10 parts per million.

Oxygen also poses a potential threat to the hydroformylation process. Once present in the reaction mixture oxygen may cause the decomposition of the rhodium-organobisphosphite catalyst in several ways. First, it may directly react with the catalyst to form oxidized rhodium species, such as rhodium oxide, or it may react with free organobisphosphite to form an organobisphosphite oxide. Such species are incapable of forming useful hydroformylation catalysts and therefore these contaminants increase the costs associated with producing olefins by the hydroformylation process. Additionally, many olefinically unsaturated compounds undergo oxidation in the presence of oxygen to form organic peroxides. These peroxides react in a similar fashion with the rhodium-organophosphite catalyst and free organobisphosphite, and therefore form a similar threat to the efficiency of the hydroformylation process.

Elemental oxygen is usually present in the syn-gas employed in the hydroformylation process. It may be removed by passing said syn-gas through a column of active metal catalyst. Such metal catalysts may include copper type catalysts, and such catalysts appear under the trade names BASF R-3-11 and BASF R-3-15.

Removing or at least reducing the amount of peroxide compounds in the olefinically unsaturated compound is preferably done as for example described in EP-A-662468, the description of which is incorporated as reference.

Of course, it is understood that any combination of the above purification techniques for the removal of the oxygen and peroxides may be employed so long as the total concentration of oxygen within the reaction mixture does not exceed 100 parts per million. More preferably, the concentration of oxygen within the reaction mixture does not exceed 50 parts per million, and still more preferably it does not exceed 10 parts per million. Likewise, the concentration of peroxides within the reaction mixture should not exceed 100 parts per million. More preferably, the concentration of peroxides does not exceed 50 parts per million, and still more preferably it does not exceed 10 parts per million.

Other unsaturated compounds present as contaminants within the olefinically unsaturated compound also pose a serious threat to the stability of the rhodium-organobisphosphite catalyst. Such compounds react with the rhodium-organobisphosphite catalyst or catalyst precursor to produce rhodium complexes which do not catalyze the desired hydroformylation reaction. Therefore catalyst activity is reduced as a result these impurities, increasing the cost of the hydroformylation process. Especially problematic unsaturated contaminants encountered within the hydroformylation process include compounds containing more than one unsaturated group such as conjugated alkadiene, alkatrienes, alkapolyenes. Typical impurities include the following compounds: butadiene, e.g. 1,3-butadiene, octatriene, e.g. 1,3,7-octatriene, vinyl cyclohexene. These compounds act as a type of poison towards the metal center by complexing to it through one or more unsaturated functionalities. The rhodium complexes which form as the result of this process do not efficiently catalyze the hydroformylation of the desired olefin. Consequently, the presence of these impurities effectively removes a source of rhodium metal from the hydroformylation process. Without wishing to be bound to any exact theory or mechanistic discourse, the decreased catalytic activity of the rhodium-phosphite complex observed in the presence of these unsaturated impurities most likely occurs as the result of the formation of coordinatively saturated rhodium complexes. These complexes lack a means to the preferred the mono-alkene carbon monoxide, or hydrogen substrates under typical hydroformylation conditions.

Thus, one objective of this invention is to prevent these unsaturated impurities present within the olefinically unsaturated compound from entering the hydroformylation process and reacting with the rhodium-organobisphosphite catalyst or catalyst precursor. This objective may be achieved by using one or more distillation procedures prior to the use of the olefinically unsaturated compound in the hydroformylation process. Distillation separates the desired olefinically unsaturated compound from the harmful unsaturated impurities by exploiting the differences in the boiling points of these two compounds. This technique is well known in the art and may be accomplished by any conventional technique. Extensive discussion of this technique can be found in e.g. Perry's Handbook of Chemical Engineering, 50^{th} edition or Grundoperationen, Lehrbuch der Technischen Chemie, Band 2, J. Gmehling, A. Brehm or Ullmann's Encyclopedia of Industrial Chemistry, Vol. B3, Unit Operations II, 5^{th} Ed., the entire disclosure of which is incorporated herein by reference thereto.

Other undesired compounds are unsaturated alkene compounds with tertiary carbon atom adjacent to the alkene functionality like for example, methyl-2-methyl-2-butenoate. Methyl-2-methyl-2-butenoate has proven to be a particularly troublesome impuritiy present in the hydroformylation of methyl-3-pentenoate. Methyl-2-methyl-butenoate, which contains a tertiary carbon atom within the alkene functionality, does not hydroformylate to a substantial extent and as a result, accumulates in the reaction mixture over time.

It is also understood that the present invention may remove unsaturated impurities from the olefinically unsaturated compounds using one or more of the above techniques, or combination thereof. One of the objects of this invention is the removal of harmful unsaturated impurities. By removal it is understood to mean that the following species are present in no greater than the following concentrations: methyl-2-methyl-2-butenoate, 2 per cent by weight; vinyl cyclohexene, 1000 ppm; butadiene, 25 ppm; other multi-unsaturated conjugated organic compounds, 25 ppm. More preferably these species are present in no greater than the following concentrations: methyl-2-methyl-2-butenoate, 1 per cent by weight; vinyl cyclohexene, 500 ppm; butadiene, 10 ppm; other multi-unsaturated conjugated organic compounds, 10 ppm. Still more preferably, these compounds are present in a concentration no greater than: methyl-2-methyl-2-butenoate, 0.5 per cent by weight; vinyl cyclohexene, 100 ppm; butadiene, 1 ppm; other multi-unsaturated conjugated organic compounds, 1 ppm. The term "per cent by weight", as used herein, is understood to mean the ratio of the weight of the impurity to the total weight of the reaction mixture, said ratio being expressed as a percentage.

Of course, it is understood that any one or more of the above purification techniques may be used in combination to achieve the desired removal of oxygen, water, peroxides, primary alcohols, and unsaturated impurities. Any combination of these techniques may be used so long as the reaction mixture contains no more than: methyl-2-methyl-2-butenoate, 2 per cent by weight; vinyl cyclohexene, 1000 ppm; butadiene, 25 ppm; other multi-unsaturated conjugated organic compounds, 25 ppm; 100 parts per million water. More preferably, the present invention will remove these impurities so that they are present within the reaction mixture in no more than: methyl-2-methyl-2-butenoate, 1 per cent by weight; vinyl cyclohexene, 500 ppm; butadiene, 10 ppm; other multi-unsaturated conjugated organic compounds, 10 ppm; 50 parts per million water; 50 parts per million alcohols; 50 parts per million peroxide; and 50 parts per million oxygen. Still more preferably, these compounds are present within the reaction mixture in no more than methyl-2-methyl-2-butenoate, 0.5 per cent by weight; vinyl cyclohexene, 100 ppm; butadiene, 1 ppm; other multi-unsaturated conjugated organic compounds, 1 ppm; 10 parts per million water; 10 parts per million alcohols; 10 parts per million peroxide; and 10 parts per million oxygen.

Illustrative rhodium-bisphosphite complex catalysts employable in such hydroformylation reactions encompassed by this invention may include those disclosed in the above mentioned patents wherein the bisphosphite ligand is a ligand selected from the class consisting of Formulas (I), (II) and (III) above. In general, such catalysts may be preformed, or formed in situ, as described e.g., in said U.S. Pat. Nos. 4,668,651 and 4,769,498, and consist essentially of rhodium in complex combination with the organobisphosphite ligand. It is believed that carbon monoxide is also present and complexed with the rhodium in the active species. The active catalyst species may also contain hydrogen directly bonded to the rhodium.

As noted above illustrative organobisphosphite ligands that may be employed as the bisphosphite ligand complexed to the rhodium catalyst and/or any free bisphosphite ligand (i.e. ligand that is not complexed with the rhodium metal in the active complex catalyst) in such hydroformylation reactions encompassed by this invention include those of Formulas (I), (II), and (III) above.

Illustrative divalent radicals represented by R¹ in the above bisphosphite formulas (I), (II) and (III) include substituted and unsubstituted radicals selected from the group consisting of alkylene, alkylene-(Q)ₙ-alkylene, phenylene, naphthylene, phenylene-(Q)ₙ-phenylene and naphthylene-(Q)ₙ-naphthylene radicals, and where Q, and n are the same as defined above. More specific illustrative divalent radicals represented by R¹ are shown by the structure of (IV) or (V) wherein (Q)ₙ is the same as above. These include, 1,1 'biphenyl-2,2'-diyl, 3,3'-dialkyl-1,1'-biphenyl-2,2'-diyl, 3,3'-dicarboxy ester-1,1'-biphenyl-2,2'-diyl, 1,1'binaphthyl-2,2'-diyl, 3,3'-dicarboxy ester-1,1'-binaphthyl-2,2'-diyl, 3,3'-dialkyl-1,1'-binaphthyl-2,2'-diyl, 2,2'-binaphthyl- 1,1'-diyl, phenylene-CH₂-phenylene, phenylene-O-phenylene, phenylene-CH(CH₃)-phenylene radicals, and the like.

Illustrative radicals represented by Z¹, Z², Z³, Z⁴, Z⁵, Z⁶, Z⁷, Z⁸, Z⁹, Z¹⁰, and Z¹¹ in above Formulas (IV) to (IX), in addition to hydrogen, include any of those organic substituents containing from 1 to 18 carbon atoms, disclosed in U.S. Pat. No. 4,668,651, or any other radical that does not unduly adversely effect the process of this invention. Illustrative radicals and substituents encompass alkyl radicals, including primary, secondary and tertiary alkyl radicals such as methyl, ethyl n-propyl, isopropyl, butyl, sec-butyl, t-butyl, neo-pentyl, n-hexyl, amyl, sec-amyl, t-amyl, iso-octyl, decyl, octadecyl, and the like; aryl radicals such as phenyl, naphthyl and the like; aralkyl radicals such as benzyl, phenylethyl, triphenylmethyl, and the like; alkaryl radicals such as tolyl, xylyl, and the like; condensated aryl radicals such as phenylene, naphthylene, and the like, alicyclic radicals such as cyclopentyl, cyclohexyl, 1-methylcyclohexyl, cyclooctyl, cyclohexylethyl, and the like; alkoxy radicals such as methoxy, ethoxy, propoxy, t-butoxy― OCH₂CH₂OCH₃, ―O(CH₂CH₂)₂OCH₃, ―O(CH₂CH₂)₃OCH₃, and the like; aryloxy radicals such as phenoxy and the like; as well as silyl radicals such as― Si(CH₃)₃, ―Si(OCH₃)₃, ―Si(C₃H₇)₃, and the like; amino radicals such as― NH₂, ―N(CH₃)₂, ―NHCH₃, ―NH(C₂H₅), and the like; acyl radicals such as― C(O)CH₃, ―C(O)C₂H₅, ―C(O)C₆H₅, and the like; carbonyloxy radicals such as ―C(O)OCH₃, ―C(O)OCH(CH₃)₂ ―(C(O)CH(CH₃)C₈H_{17,} and the like; oxycarbonyl radicals such as ―O(CO)C₆H₅, and the like; amido radicals such as ―CONH₂, ―CON(CH₃)₂, ―NHC(O)CH₃, and the like; sulfonyl radicals such as― S(O)₂C₂H₅ and the like; sulfinyl radicals such as ―S(O)CH₃ and the like; thionyl radicals such as ―SCH₃, ―SC₂H₅, ―SC₆H₅, and the like; phosphonyl radicals such as ―P(O)(C₆H₅)₂, ―P(O)(CH₃)₂, ―P(O)(C₂H₅)₂, ―P(O)(C₃H₇)₂, P(O)CH₃(C₆H₅), ―P(O)(H)(C₆H₅), and the like.

Illustrative radicals represented by X¹, X², X³, X⁴, X⁵ and X⁶ in above Formulas (IV) to (IX) include those illustrated and discussed above as representing Z¹ to Z¹¹, except hydrogen and condensated aryl radicals.

Illustrative radicals represented by Y¹, Y², Y³, Y⁴ and Y⁵ in above Formulas (IV) to (IX) include those illustrated and discussed above as representing Z¹ to Z¹¹, except condensated aryl radicals.

More preferably, X¹ is the same as X² and Z¹ is the same as Z² in Formula (IV), X³ is the same as X⁴, Z³ is the same as Z⁴, and Y¹, Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

Specific illustrative examples of the bisphosphite ligands employable in this invention include such preferred ligands as:
3,3'-bis(carboxyisopropyl)-1,1'-binaphthyl-2,2'-diyl-bis[bis(1-naphthyl)]phosphite having the formula: 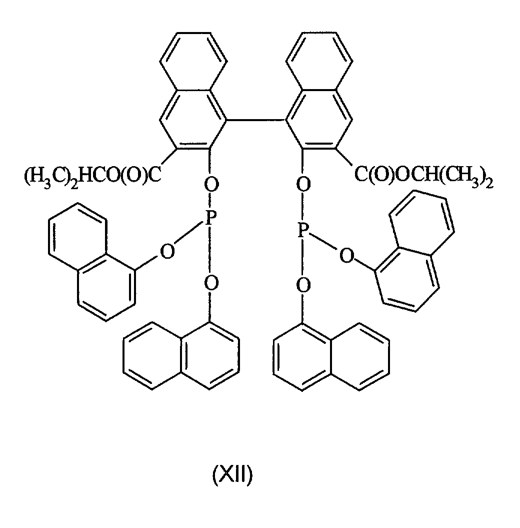
[3,3'-bis(t-butyl)-5,5'-dimethoxy-1,1'-biphenyl-2,2'-diyl]-bis(oxy)]-bis(dibenzo[d,f] [1,3,2])dioxaphosphepin having the formula: 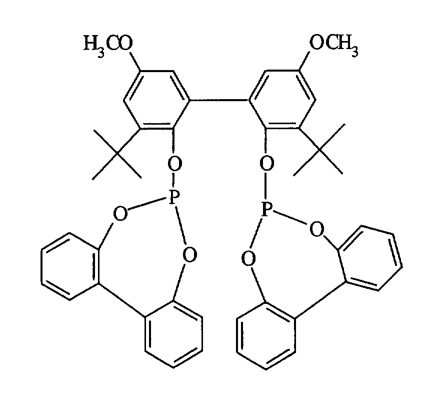
3,3'-bis(carboxyisopropyl)-1,1'-binaphthyl-2-yl-bis[(1-naphthyl)]phosphite-2'-yl-oxy-dibenzo[d,f] [1,3,2]dioxaphosphepin having the formula: 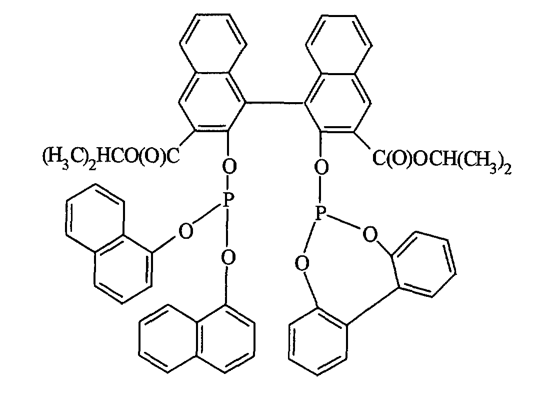
5,5'-bis(t-butyl)-3,3'-dimethoxy-1,1'-biphenyl-2,2'-diyl-bis[bis(1-naphthyl)]phosphite having the formula: 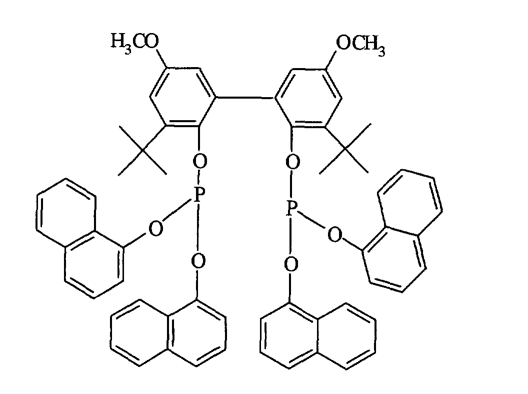
3,3'-bis(carboxymethyl)-1,1'-binaphthyl-2,2'-diyl-bis[bis(2-t-butylphenyl)]phosphite having the formula: 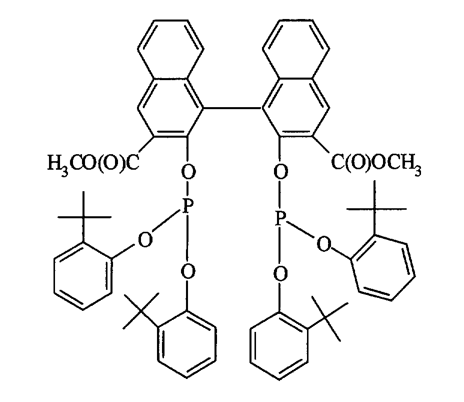
[3,3'-bis(t-butyl)-5,5'-dimethoxy-1,1'-biphenyl-2,2'-diyl]-bis(oxy)]-bis([1,1'-dinaphto[d,f] [1,3,2])dioxaphosphepin having the formula: 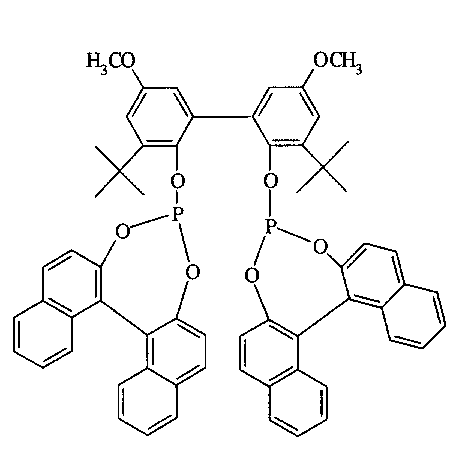
and the like.

Such types of bisphosphite ligands employable in this invention and/or methods for their preparation are well known as seen disclosed for example in U.S. Pat. Nos. 4,668,651; 5,288,918; 5,710,306, the entire disclosure of which is incorporated herein by reference thereto.

In general, such hydroformylation reactions involve the production of aldehydes by reacting an olefinic compound with carbon monoxide and hydrogen in the presence of a rhodium-organobisphosphite complex catalyst in a liquid medium that also contains a solvent for the catalyst. The process may be carried out in a continuous single pass mode or more preferably in a continuous liquid catalyst recycle manner. The recycle procedure generally involves withdrawing a portion of the liquid reaction medium containing the catalyst and aldehyde product from the hydroformylation reaction zone, either continuously or intermittently, and distilling the aldehyde product therefrom in one or more stages, in a separate distillation zone in order to recover the aldehyde product and other volatile materials in vaporous form, the non-volatilized rhodium catalyst containing residue being recycled to the reaction zone. Likewise, the recovered non-volatilized rhodium catalyst containing residue can be recycled with or without further treatment to the hydroformylation zone in any conventional manner desired. Accordingly, the processing techniques of this invention may correspond to any known processing techniques such as heretofore employed in conventional liquid catalyst recycle hydroformylation reactions.

As noted above the hydroformylation reaction conditions that may be employed in the hydroformylation processes encompassed by this invention may include any suitable continuous hydroformylation conditions heretofore disclosed in the above-mentioned patents. Further, the hydroformylation process may be conducted at a reaction temperature from about 25° C. to about 150°. In general hydroformylation reaction temperature of about 70° C. to about 120° are preferred for all types of olefinic starting materials, the more preferred reaction temperatures being from about 90° C. to about 100° C. and most preferably about 95° C.

The olefinic starting material reactants that may be employed in the hydroformylation reactions encompassed by this invention include olefinic compounds containing from 2 to 30 carbon atoms. Such olefinic compounds can be terminally or internally unsaturated and be of straight-chain, branched chain or cyclic structures, as well as be olefin mixtures, such as obtained from the oligomerization of propene, butene, isobutene, etc., (such as so called dimeric, trimeric or tetrameric propylene, and the like, as disclosed, e.g., in U.S. Pat. Nos. 4,518,809 and 4,528,403). Moreover, mixtures of two or more different olefinic compounds may be employed as the starting hydroformylation material if desired. Further such olefinic compounds and the corresponding aldehyde products derived therefrom may also contain one or more groups or substituents which do not unduly adversely affect the hydroformylation process or the process of this invention such as described, e.g., in U.S. Pat. Nos. 3,527,809; 4,668,651 and the like.

Illustrative olefinic unsaturated compounds are alpha-olefins, internal olefins, alkyl alkenoates such as methyl-2-pentenoate, methyl-3-pentenoate and methyl-4-pentenoate, alkenyl alkanoates, alkenyl alkyl ethers, alkenols, and the like, e.g., ethene, propene, 1-butene, 1-pentene, 1-hexene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 2-butene, 2-methyl propene (isobutylene), 2-methylbutene, 2-pentene, 2-hexene, 3-hexene, 2-heptene, cyclohexene, 2-ethyl-1-hexene, 2-octene, allyl alcohol, allyl butyrate, hex-1-en-4-ol, oct-1-en-4-ol, vinyl acetate, allyl acetate, 3-butenyl acetate, vinyl propionate, allyl propionate, methyl methacrylate, vinyl ethyl ether, vinyl methyl ether, allyl ethyl ether, n-propyl-7-octenoate, 3-butenenitrile, 3-pentenenitrile and 5-hexenamide.

Of course, it is understood that mixtures of different olefinic starting materials can be employed, if desired, by the hydroformylation process of the subject invention. More preferably the subject invention is especially useful for the production of aldehydes, by hydroformylating alpha olefins containing from 2 to 20 carbon atoms, including isobutylene, and internal olefins containing from 4 to 20 carbon atoms as well as starting material mixtures of such alpha olefins and internal olefins. Still more preferably the subject invention is especially useful for the production of aldehydes from methyl-3-pentenonate, in any isomeric form, or mixture of isomeric forms.

As noted above the hydroformylation reaction conditions that may be employed in the hydroformylation processes encompassed by this invention may include any suitable continuous hydroformylation conditions heretofore disclosed in the above-mentioned patents. For instance, the total gas pressure of hydrogen, carbon monoxide and olefinic unsaturated starting compound of the hydroformylation process may range from about 1 to about 10,000 psia. In general, however, it is preferred that the process be operated at a total gas pressure of hydrogen, carbon monoxide and olefinic unsaturated starting compound of less than about 1500 psia and more preferably less than about 500 psia. The minimum total pressure being limited predominately by the amount of reactants necessary to obtain a desired rate of reaction. More specifically the carbon monoxide partial pressure of the hydroformylation process of this invention is preferable from about 1 to about 120 psia, and more preferably from about 3 to about 90 psia, while the hydrogen partial pressure is preferably about 15 to about 160 psia and more preferably from about 30 to about 100 psia. In general H₂:CO molar ratio of gaseous hydrogen to carbon monoxide may range from about 1:10 to 100:1 or higher, the more preferred hydrogen to carbon monoxide molar ratio being from about 1:10 to about 10:1. The term syn-gas as used herein is understood to mean any gaseous mixture comprised of hydrogen and carbon monoxide.

As noted above, the continuous hydroformylation process of this invention involves the use of a rhodium-organobisphosphite ligand complex catalyst as described herein. Of course mixtures of such catalysts can also be employed if desired. The amount of rhodium-phosphite complex catalyst present in the reaction medium of a given hydroformylation process encompassed by this invention need only be that minimum amount necessary to provide the given rhodium concentration desired to be employed and which will fumish the basis for at least the catalytic amount of rhodium necessary to catalyze the particular hydroformylation process involved such as disclosed e.g. in the above-mentioned patents. In general, rhodium concentrations in the range of from about 10 ppm to about 1000 ppm, calculated as free rhodium, in the hydroformylation reaction medium should be sufficient for most processes, while it is generally preferred to employ from about 10 to 500 ppm of rhodium and more preferably from 25 to 350 ppm to rhodium.

In addition to the rhodium-organobisphosphite catalyst the hydroformylation process encompassed by this invention may be carried out in the presence of free organobisphosphite ligand, i.e. ligand that is not complexed with the rhodium metal of the complex catalyst employed. Said free organobisphosphite ligand may correspond to any of the above defined organobisphosphite ligands discussed above as employable herein. When employed it is preferred that the free organobisphosphite ligand be the same as the organobisphosphite ligand of the rhodium-organobisphosphite complex catalyst employed. However, such ligands need not be the same in any given process. Moreover, while it may not be absolutely necessary for the hydroformylation process to be carried out in the presence of any such free organobisphosphite ligand, the presence of at least some amount of free organobisphosphite ligand in the hydroformylation reaction medium is preferred. Thus the hydroformylation process of this invention may be carried out in the absence or presence of any amount of free organobisphosphite ligand, e.g. up to 100 moles, or higher per mole of rhodium metal in the hydroformylation reaction medium. Preferably the hydroformylation process of this invention is carried out in the presence of from about 1 to about 50 moles of organobisphosphite ligand, and more preferably from about 1 to about 4 moles of organobisphosphite ligand, per mole of rhodium metal present in the reaction medium; said amounts of organobisphosphite ligand being the sum of both the amount of organobisphosphite ligand that is bound (complexed) to the rhodium metal present and the amount of free (non-complexed) organobisphosphite ligand present. Of course, if desired, make-up or additional organobisphosphite ligand can be supplied to the reaction medium of the hydroformylation process at any time and in any suitable manner, e.g. to maintain a predetermined level of free ligand in the reaction medium.

The hydroformylation reactions encompassed by this invention may also be conducted in the presence of an organic solvent for the rhodium-organobisphosphite complex catalyst and any free organobisphosphite ligand that might be present. Any suitable solvent which does not unduly adversely interfere with the intended hydroformylation process can be employed. Illustrative suitable solvents for rhodium catalyzed hydroformylation processes include those disclosed e.g. in U.S. Pat. No. 4,668,651. Of course mixtures of one or more different solvents may be employed if desired. Most preferably the solvent will be one in which the olefinic starting material, and catalyst, are all substantially soluble. In general, it is preferred to employ aldehyde compounds corresponding to the aldehyde products desired to be produced and/or higher boiling aldehyde liquid condensation by-products as the primary solvent, such as the higher boiling aldehyde liquid condensation by-products that are produced in situ during the hydroformylation process. Indeed, while one may employ any suitable solvent at the start up of a continuous process, the primary solvent will normally eventually comprise both aldehyde products and higher boiling aldehyde liquid condensation by-products due to the nature of such continuous processes. Such aldehyde condensation by-products can also be preformed if desired and used accordingly. Of course, the amount of solvent employed is not critical to the subject invention and need only be that amount sufficient to provide the reaction medium with the particular rhodium concentration desired for a given process. In general, the amount of solvent may range from 0 percent by weight up to about 95 percent by weight or more based on the total weight of the reaction medium.

The distillation and separation of the desired aldehyde product from the rhodium-bisphosphite complex catalyst containing product solution may take place at any suitable temperature desired. In general it is recommended that such distillation take place at low temperatures, such as below 150° C., and more preferably at a temperature in the range of from about 50° C. to about 130° C., and most preferably between 70 and 115° C. It is also generally recommended that such aldehyde distillation takes place under reduced pressure, e.g. a total gas pressure that is substantially lower than the total gas pressure employed during hydroformylation when low boiling aldehydes (e.g. C₄ to C₆) are involved or under vacuum when high boiling aldehydes (e.g. C₇ or greater) are involved. For instance, a common practice is to subject the liquid reaction product medium removed from the hydroformylation reactor to a pressure reduction so as to volatilize and remove a substantial portion of the unreacted gases dissolved in the liquid medium and then pass said liquid medium which now contains a much lower syn gas concentration than was present in the hydroformylation reaction medium to the distillation zone e.g. vaporizer/separator, wherein the desired aldehyde product is distilled. In general distillation pressures ranging from vacuum pressures or below on up to total gas pressure of about 50 psig should be sufficient for most purposes.

Of course it is to be understood that while the optimization of the subject invention necessary to achieve the best results and efficiency desired are dependent upon one's experience in the utilization of the subject invention, only a certain measure of experimentation should be necessary to ascertain those conditions which are optimum for a given situation and such should be well within the knowledge of one skilled in the art and easily obtainable by following the more preferred aspects of this invention as explained herein and/or by simple routine experimentation.

Finally, the aldehyde products of the hydroformylation process of this invention have a wide range of utility that is well known and documented in the prior art e.g. they are especially useful as starting materials for the production of alcohols and acids, as well as for the production of monomeric and polymeric compounds such as £-caprolactam, adipic acid, nylon-6 and nylon-6,6.

The following examples are illustrative of the present invention and are not be regarded as limitive. It is to be understood that all of the parts, percentages and proportions referred to herein and in the appended claims are by weight unless otherwise indicated.

### EXAMPLE 1

This example illustrates that lower water contamination leads to lower consumption of costly bisphosphite ligand.

In a continuous process, a mixture of 89 wt% methyl-3-pentenoate and 11 wt % trans-methyl-2-pentenoate was hydroformylated using a Rh/naftol-3 (with structure XII) catalyst. The reaction temperature and pressure were 95°C and 5 bara. The molar ratio of H₂ and CO was 1:1. The average liquid hold-up time in the stirred continuos tank reactor was 4 hours. A rhodium concentration of 150 ppm was applied. Using a ligand make-up stream, a slight molar excess of the naftol-3 ligand was constantly maintained. The liquid reactor effluent was flashed to atmospheric pressure to remove a large part of the dissolved gasses. After this flash, the liquid was cooled and passed over a dry weakly basic ion exchange resin to remove acidic ligand degradation products. Subsequently the mixture was passed to a vacuum evaporator where most of the unconverted substrates and the aldehyde products were removed as overheads. The bottom stream, containing the non-volatile catalyst, was continuously recycled to the reactor.

The feed olefin feedstock of methyl-3-pentenoate/trans-methyl-2-pentenoate was purified by batch distillation. The forerun containing lights such as water was discarded and the purified heart cut was used in the hydroformylation reaction step.

During the first seven days of the continuous run, anhydrous methyl-3-pentenoate/trans-methyl-2-pentenoate was applied. The average ligand consumption during this period was 110 mg Naftol-3/kg methyl-5-formylvalerate product. After 7 days 600 ppm water was deliberately added to the methyl-3-pentenoate/trans-methyl-2-pentenoate feed and the unit was run for 2 more days. The ligand consumption rate tripled to 340 mg naftol-3 per kg methyl-5-formylvalerate.

### EXAMPLE 2

This example illustrates that the hydroformylation rate with feeds free from multi-unsaturated compounds such as butadiene is much higher than with a feed contaminated with these compounds. The conjugated alkadienes act as reversible catalyst poisons.

In a continuous process, a mixture of methyl pentenoates was hydroformylated using a Rh/naftol-3 catalyst. The reaction temperature and pressure were 95 ° C and 5 bara. The molar ratio of H₂ and CO was 1:1. The average total liquid hold-up time in three stirred continuous tank reactors in series was 6 hours. A rhodium concentration of 100 ppmw was applied. Using a ligand make-up stream to the first reactor, a molar excess of the naftol-3 ligand to Rh was constantly maintained.

The liquid reactor effluent was flashed to atmospheric pressure to remove a large part of the dissolved gasses. After this flash, the liquid was passed across a dry weakly basic ion exchange resin to remove acidic ligand degradation products. Then the liquid was fed to a vacuum evaporator where most of the unconverted substrates and the aldehyde products were removed as overheads. The bottom stream from the evaporator, containing the non-volatile catalyst, was continuously recycled to the reactor. The overhead products from the evaporator were removed from the process and analyzed by gas chromatography.

In a continuous run, methyl-3-pentenoate contaminated with low amounts (50 ppmw) of multi-unsaturated hydrocarbons such as 1,3-butadiene (the boiling point of the contaminants < boiling point of methyl-3-pentenoate) was fed to the process described above. The catalyst showed negligible activity. Only when the contaminated methyl-3-pentenoate was replaced by non-contaminated methyl-3-pentenoate, the catalyst activity slowly recovered to the normal value of STY= 0.65 mole M5FV/Itr.hr by slow displacement of the reversible poison from the catalyst complex.

### EXAMPLE 3

This example illustrates that the hydroformylation rate with a low reactor concentration of methyl-2-methyl-2-butenoate is significantly higher than with a high M2M2B-concentration. This effect is due to to high accumulation in the M3P/Mt2P recycle and reactor section in a continuous process.

In a continuous process, a mixture of methyl pentenoates and 2 wt% methyl-2-methyl-2-butenoate was hydroformylated using a rhodium/naftol-3 catalyst. The reaction temperature and pressure were 95°C and 5 bara. The molar ration of H₂ and CO was 1:1. The average total liquid hold-up time in three stirred continuous tank reactors in series was 6 hours. A rhodium concentration of 100 ppmw was applied. Using a ligand make-up stream to the first reactor, a molar excess of the naftol-3 ligand to rhodium was constantly maintained.

The liquid reactor effluent was flashed to atmospheric pressure to remove a large part of the dissolved gasses. After this flash, the liquid was passed across a dry weakly basic ion exchange resin to remove acidic ligand degradation products. Then the liquid was fed to a vacuum evaporator where most of the unconverted substrates and the aldehyde products were removed as overheads.

The bottom stream from the evaporator, containing the non-volatile catalyst, was continuously recycled to the reactor. The overhead product from the evaporator was subsequently split by distillation into a low boiling fraction containing methyl valerate and methyl pentenoates and a high boiling fraction containing methyl formylvalerates. The methyl formylvalerates fraction was removed from the process. The light fraction was split again by distillation into a heavier fraction containing mainly M3P and Mt2P that was recycled to the reactor section. This heavier M3P/Mt2P-fraction also contains more than 95% of the M2M2B in the feed to the second destillation. The lighter fraction with compounds lighter than M3P (mainly methyl valerate, methyl-4-pentenoate, methyl-cis-2-pentenoate) was discarded from the process.

The continuous process was operated for 550 hours with recycle of the catalyst and unconverted methyl-3-pentenoate/methyl-trans-2-pentenoate. After 550 hours, the methyl-2-methyl-2-butenoate concentration in the reactors had climbed from initially less than 2 wt % to 13 wt % and the methyl-2-methyl-2-butenoate concentration in the methyl-3-pentenoate/methyl-trans-2-pentenoate recycle stream had climbed to more than 50 wt%, whereas the feed only contained 2 wt% of methyl-2-methyl-2-butenoate. Clearly, methyl-2-methyl-2-butenoate was accumulating excessively in the process due to a very low hydroformylation rate. This was confirmed by the hydroformylation rate constants determined by regression:
Rate constant (M2M2B): 1.17 x 10⁻⁴ /hr/ppm Rh
Rate constant (M3P): 5.4 x 10⁻³ /hr/ppm Rh
Due to the accumulation of methyl-2-methyl-2-butenoate the reactor productivity (STY - Space Time Yield) fell from 0.66 mole methyl-5-formylvalerate/Itr.hr at <2 wt% methyl-2-methyl-2-butenoate to 0.51 mole methyl-5-formylvalerate /Itr.hr at
13 wt% methyl-2-methyl-2-butenoate in the reaction liquid.

## Claims

1. A continuous process for producing an aldehyde comprising exposing an olefinically unsaturated compound to a mixture comprising carbon monoxide, hydrogen and a rhodium-organobisphosphite complex catalyst, wherein said mixture contains less than:
a) 1000 part per million vinyl cyclohexene,
b) 25 parts per million butadiene,
c) 25 parts per million of other multi-unsaturated conjugated organic compounds,
d) 100 parts per million water,
e) 100 parts per million peroxide,
f) 2 percent by weight methyl-2-methyl-2-butenoate,
g) 100 parts per million primary alcohol,
h) 100 parts per million oxygen, and
i) 10 parts per million halogen.

2. A process according to claim 1, wherein said mixture comprises less than:
a) 500 part per million vinyl cyclohexene,
b) 10 parts per million butadiene,
c) 10 parts per million of other multi-unsaturated conjugated organic compounds,
d) 50 parts per million water,
e) 50 parts per million peroxide,
f) 1 per cent by weight methyl-2-methyl-2-butenoate,
g) 50 parts per million primary alcohol, and
h) 50 parts per million oxygen.

3. A process according to claim 2, wherein said mixture comprises less than:
a) 100 part per million vinyl cyclohexene,
b) 1 parts per million butadiene,
c) 1 parts per million of other multi-unsaturated conjugated organic compounds,
d) 10 parts per million water,
e) 10 parts per million peroxide,
f) 0.5 per cent by weight methyl-2-methyl-2-butenoate,
g) 10 parts per million primary alcohol, and
h) 10 parts per million oxygen.

4. The process according to any one of claims 1-3, wherein the organobisphosphite ligand of said rhodium-organobisphosphite complex catalyst is a ligand selected from the class consisting of 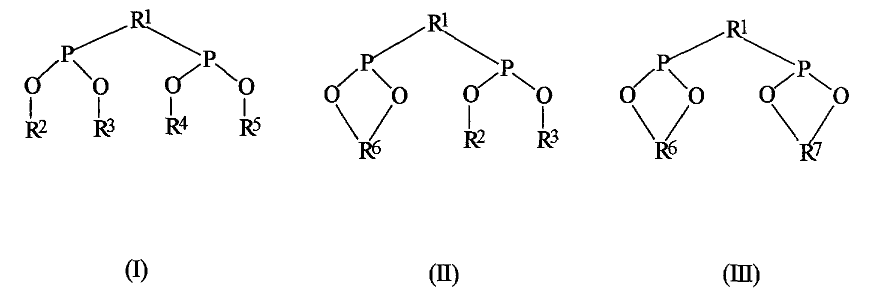 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different;
wherein each Q individually represents a divalent bridging group of ―O― or-CR'R"- wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 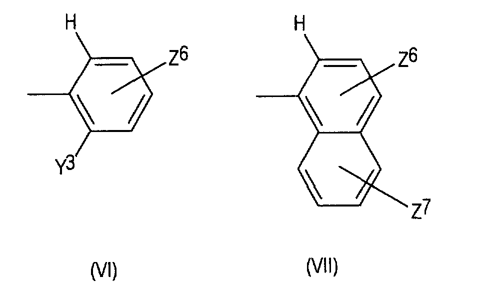 wherein R⁶ and R⁷ might be the same or different and each is individually represented by the structure of (VIII) or (IX), 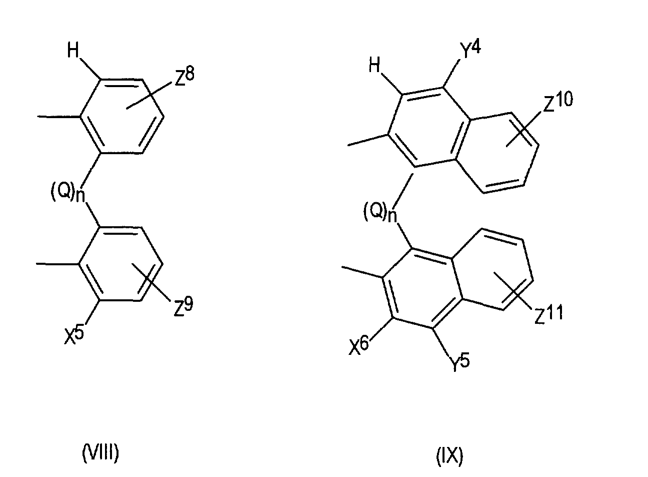 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical,
wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

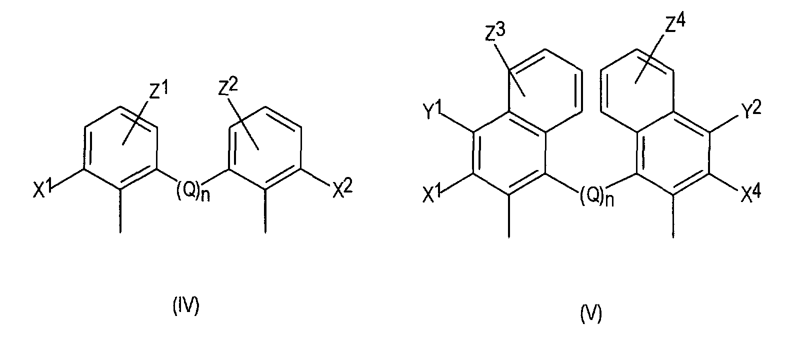

5. The process of claim 4, wherein R¹ is represented by the structure of (IV), (V), (VII), (VIII) or (IX) wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

6. The process according to any one of claims 4-5, wherein the ligand used is [3,3'-bis(t-butyl)-5,5'-dimethoxy-1,1'-biphenyl-2,2'-diyl]-bis(oxy)]-bis(dibenzo[d,f][1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

7. Process according to any one of claims 1-6, wherein the olefinically unsaturated compound is methyl-3-pentenoate or a mixture of methyl-4-, methyl-3- and methyl-2-pentenoate.

8. The process according to any one of claims 1 through 7 wherein the olefinically unsaturated compound is percolated prior to its introduction into the hydroformylation process through one or more compounds selected from the following class: alumina, silica, and molecular sieves.

9. The process according to any one of claims 1 through 8 wherein contaminating water or a contaminating primary alcohol is removed from the olefinically unsaturated compound by distillation.

10. The process according to any one of claims 1 through 9 wherein the solvent is percolated prior to its introduction into the hydroformylation process through one or more compounds selected from the following class: aluminum oxide, silica, and molecular sieves.

11. The process according to any one of claims 1 through 10 wherein said hydrogen and/or carbon monoxide is passed through a metal catalyst prior to its introduction into the hydroformylation process.
